# EUROPÄISCHE PATENTSCHRIFT

(11) **EP 4 295 821 B1**
(45) Veröffentlichungstag und Bekanntmachung des Hinweises auf die Patenterteilung: **28.05.2025**
(21) Anmeldenummer: 23020300.2
(22) Anmeldetag: 19.06.2023
(51) Int. Cl.: A61F 5/01

(54) **ORTHESE FÜR DAS DAUMENSATTELGELENK**
ORTHESIS FOR THE THUMB SADDLE JOINT
ORTHÈSE POUR L'ARTICULATION TRAPÉZO-MÉTACARPIENNE

(30) Priorität: 20.06.2022 DE 102022115336
(43) Veröffentlichungstag der Anmeldung: 27.12.2023
(73) Patentinhaber: Matzen, Miklas, 21382 Brietlingen (DE)
(72) Erfinder: Matzen, Miklas, 21382 Brietlingen (DE)
(74) Vertreter: Völger, Karl Wolfgang

(56) Entgegenhaltungen:
- WO-A1-2021/181072
- US-A1- 2016 296 359
- US-A1- 2017 333 242
- US-A1- 2018 168 844

## Beschreibung

Die vorliegende Erfindung betrifft eine Orthese für das Daumensattelgelenk zur Linderung der Beschwerden einer Daumensattelgelenkserkrankung aus dem degenerativen, entzündlichen und rheumatischen Formenkreis. Ferner betrifft die Erfindung die Verwendung dieser Orthese.

Das Daumensattelgelenk (CMP), lat. Articulatio carpometacarpalis pollicis, ist die gelenkige Verbindung zwischen dem großen Vieleckbein, lat. Os trapezium, in der Handwurzel und dem Mittelhandknochen des Daumens, lat. Os metacarpale I. Es ist das einzige Gelenk der fünf Grundgelenke, Articulationes carpometacarpales, das frei beweglich ist. Mögliche Bewegungen sind Flexion, Extension, Abduktion, Adduktion, Rotation und Opposition. Das Daumensattelgelenk (CMP) wird vor allem für die Gegenüberstellung (Opposition) des Daumens benötigt und hat daher eine zentrale Bedeutung für das Greifen.

Bei der Arthrose des Daumensattelgelenks (CMP), konkret als Rhizarthrose bezeichnet, handelt es sich um eine Erkrankung infolge derer es zu unspezifischen Abnutzungserscheinungen einhergehend mit einer Verringerung der Gleitschicht des Knorpelgelenks bis hin zu dessen vollständiger Auflösung kommen kann. Damit verbunden ist eine zunehmend schmerzhafte Entzündung und Druckempfindlichkeit. Des Weiteren wird der Kapsel-Band-Apparat sekundär geschädigt und geschwächt.

Bisher aus dem Stand der Technik bekannte Behandlungsmethoden der Rhizarthrose stellen das Daumensattelgelenk (CMP) ruhig, wodurch als schmerzhaft empfundene Bewegungen mehr oder weniger effektiv unterbunden werden. Alternativ kann ein nicht einstellbares, nur bei Beugung aktives Auseinanderziehen des Daumensattelgelenks (CMP) herbeigeführt werden.

So wird in DE 30 06 362 A1 eine Vorrichtung aus zwei offenen, am Unterarm befestigten Stegen beschrieben, bei der unter anderem der Daumen in einer zum Schienenende verlaufenden Hülse so eingeführt ist, dass die gesamte Hand einschließlich des Handgelenks bewegungsunfähig arretiert ist.

DE 35 19 493 A1 offenbart eine Vorrichtung zum Ruhigstellen des Daumensattelgelenks (CMP), bei welcher der Daumen und die Hand zumindest teilweise von einem festen Formkörper umschlossen fixiert sind. Durch die anatomische Ausformung der Schiene verbleibt die Hand in ihrer natürlichen Lage und bewirkt so die Ruhigstellung des Daumensattelgelenks (CMP) als auch des Daumengrundgelenks (MCP), lat. Articulatio metacarpophalangea.

Auch in WO 2011/110420 A1 wird eine Vorrichtung beansprucht, deren Aufgabe eine spezifische und vollständige Immobilisierung des Daumensattelgelenks (CMP) erreicht und so die schmerzhafte Bewegung der von der Arthrose beeinträchtigten Gelenke sicher unterbindet.

Die bekannten Vorrichtungen aus dem Stand der Technik haben den Nachteil, dass durch die Ruhigstellung des Gelenks der Kapsel-Band-Muskelapparat in kurzer Zeit erschlafft und infolge dessen eine fortschreitende Einschränkung in der Beweglichkeit einhergeht mit einer Verschlimmerung der Schmerzsymptome.

In DE 20 2014 103 362 U1 wird eine Vorrichtung aus Kunststoff beschrieben, welche dadurch gekennzeichnet ist, dass sie anatomisch angepasst und lösbar am Handgelenk fixiert ist. Sie verläuft lateral über den Handballen und schließt korkenzieherähnlich am ersten Daumengelenk ab. Diese Vorrichtung hat den Nachteil, dass keine Verstellmöglichkeit gegeben ist und ein Auseinanderziehen des Daumensattelgelenks (CMP) nur bei Beugung des Daumenendgelenks (IP), lat Articulatio interphalangea, erfolgt, was einen Nutzen für Patienten, welche z.B. einen Rollstuhl über die Antriebsräder selber antreiben müssen, nicht gewährleisten kann.

Weitere Nachteile der bekannten Vorrichtungen bestehen darin, dass die gewünschte Ruhigstellung nur dann erreichbar ist, wenn die mitunter auch plastisch verformbare und damit anatomisch angepasste Schiene die zu arretierenden Körperteile fest umschließt. Dadurch wird die Blutzirkulation beeinträchtigt und infolge punktuell wirkenden Drucks werden Empfindlichkeiten und Scheuerstellen hervorgerufen. Zudem wird das Ausführen auch nur leichter körperlicher Betätigung infolge der Ruhigstellung nahezu komplett unterbunden und der aus Daumen und Mittelfinger bildbare Pinzettengriff deutlich erschwert oder gar unmöglich.

In Erkenntnis dieser Probleme weicht DE 20 2012 009 952 U1 von den vorerwähnten Prinzipien ab und stellt das Daumensattelgelenk (CMP) nicht nur ruhig, sondern spreizt dieses durch eine in die anatomisch angepasste Orthese eingeklebte flügelförmige Pelotte aus einem nachgiebig gepolsterten Material. Ein Druck auf die Daumenspitze, wie er insbesondere beim Pinzettengriff wirkt, wird so über die anatomisch ausgeformte Orthese auf die Mittelhand abgeleitet, was gleichsam den Druck auf die Pelotte erhöht und die aufeinander reibenden Gelenkschalen zunehmend expandiert.

Allerdings birgt diese Vorrichtung den Nachteil, dass das Daumensattelgelenk (CMP) fest umschlossen in einer Schiene ruht. Durch den gegen die Schiene ausgelösten Druck, der durch die eingeklebte Pelotte noch erhöht wird, kann es beim Tragen der Vorrichtung zu punktuellen Belastungen bis hin zu Druckstellen kommen. Das Anpassen der Orthese bereitet darüber hinaus nicht unerhebliche Schwierigkeiten, weil die Pelotte nur exakt ausgerichtet das Auseinanderdrücken des Gelenkes bewirkt, andernfalls den Schmerz der Grunderkrankung sogar verstärkt. Die hier beschriebene Vorrichtung ist zudem nur wirksam, wenn das Daumenendgelenk (IP) bewegt wird.

Angesichts der vorstehend beschriebenen Nachteile und Probleme des Standes der Technik ist es Ziel der vorliegenden Erfindung, die vorstehenden Nachteile zu überwinden und eine Orthese bereitzustellen, durch die das arthrotisch veränderte Daumensattelgelenk (CMP) dynamisch entlastet wird, bei gleichzeitiger Forderung des Kapsel-Band-Muskelapparats.

Diese Aufgabe wird erfindungsgemäß in einem ersten Aspekt der vorliegenden Erfindung durch eine Orthese (1) für das Daumensattelgelenk (CMP) gelöst, umfassend
- eine Schale (101), die vom proximalen Handwurzelgelenk (RP) nach distal bis zum Daumengrundgelenk (MCP) verläuft, wobei die Schale (101) an ihrem distalen Ende (im Bereich des Daumengrundgelenks) sowohl offen als auch zirkulär vollkommen umschließend ausführbar ist,
- einen an der Schale (101) befestigten Verschluss (103) im Bereich des proximalen Handwurzelgelenks (RP), welcher am proximalen Handwurzelgelenk (RP) zirkulär vollkommen umschließend fixierbar ist,
- einen in der Schale (101) vorgesehenen Kanal (111), welcher mit dem Kanal-Ausgang (117) endet,
- eine flexible Schiene (113), die teilweise in dem Kanal (111) von der Schale (101) bis zum Daumenendgelenk (IP) verläuft und diesen durch den Kanal-Ausgang (117) verlässt,
- eine Halterung (109), die an einem ersten Ende der flexiblen Schiene (113) vorgesehen ist und am distalen Ende des ersten Daumenknochens (PPI) zirkulär vollkommen umschließend fixierbar ist, und
- ein Stellelement (115), das an der Schale (101) angeordnet ist und das die flexible Schiene (113) an deren zweitem Ende reversibel aufnimmt,
dadurch gekennzeichnet, dass die flexible Schiene (113) von dem Stellelement (115) durch den Kanal (111) verläuft und diesen lateral/proximal des Daumengrundgelenks (MCP) verlässt, wobei die flexible Schiene (113) im Bereich des Kanal-Ausgangs (117) parallel zum anatomischen Gelenkdrehpunkt verläuft und die flexible Schiene (113) die Schale (101) mit der Halterung (109) reversibel lösbar verbindet.

Die erfindungsgemäße Orthese (1) ist zur Befestigung an einer menschlichen Hand vorgesehen. Abhängig von der rechten oder der linken Hand ist die erfindungsgemäße Orthese (1) spiegelbildlich ausgebildet.

Die Schale (101) wird in ihrer konkreten Form individuell an die Hand des Patienten angepasst und liegt am Handballen passgenau an. Sie verläuft vom proximalen Handwurzelgelenk (RP) nach distal bis zum Daumengrundgelenk (MCP) wobei die Schale (101) an ihrem distalen Ende (im Bereich des Daumengrundgelenks) sowohl offen als auch zirkulär vollkommen umschließend gestaltet werden kann.

Der Verschluss (103) im Bereich des proximalen Handwurzelgelenks (RP), lat. Articulatio radiocarpalis, welcher am proximalen Handwurzelgelenk (RP) zirkulär vollkommen umschließend fixierbar ist, kann in der einfachsten Ausführungsform aus einem festen Band bestehend, das um das Handgelenk geschlungen wird. Des Weiteren ist ein Verschluss (103) mit einem Klett-/Flauschband mit einem Umlenker erfindungsgemäß möglich.

Die flexible Schiene (113) dient einerseits dazu, die Halterung (109) für das distale (körperferne) Ende des ersten Daumenknochens (PPI), lat. Phalanx proximalis I, aufzunehmen. Andererseits erstreckt sich die flexible Schiene (113) in etwa parallel zum ersten Daumenknochens (PPI). Der Begriff "Schiene" umfasst jegliches langgestreckte Bauteil, das dazu geeignet ist, diese Funktion zu übernehmen. Die flexible Schiene (113) im Sinne der vorliegenden Erfindung schließt daher auch Stäbe, Rohre und dergleichen ein. Diese können aus verschiedenen Materialen bestehen, welche unterschiedliche Grade der Flexibilität und/oder Festigkeit aufweisen können.

Die Halterung (109) umschließt das distale Ende des ersten Daumenknochens (PPI) zirkulär vollkommen und ist damit reversibel fixierbar. In der einfachsten Ausführungsform kann auch diese Halterung aus einem schalenförmigen Teil und einem festen Band bestehen, das um den Daumen geschlungen wird. Des Weiteren ist ein Verschluss mit einem Klett-/Flauschband mit einem Umlenker erfindungsgemäß möglich.

Die vorliegende Erfindung zeichnet sich dadurch aus, dass lateral (d.h. auf der Höhe) des Daumengrundgelenks (MCP) eine flexible Schiene (113) von dem Stellelement (115) durch den Kanal (111) verläuft und diesen lateral/proximal des Daumengrundgelenks (MCP) verlässt, wobei die flexible Schiene (113) im Bereich des Kanal-Ausgangs (117) parallel zum anatomischen Drehpunkt liegt. Durch diese Anordnung der flexiblen Schiene (113) wird die Beweglichkeit der Hand, bzw. des Daumens trotz anlegender Orthese (1) gewährleistet. Ferner zeichnet sich die erfindungsgemäße Orthese (1) dadurch aus, dass die flexible Schiene (113) die Schale (101) mit der Halterung (109) reversibel lösbar verbindet.

Die Formulierung "reversibel lösbar" bedeutet, dass die flexible Schiene (113) in dem Stellelement (115) so aufgenommen ist, dass sie sowohl befestigt als auch wieder gelöst werden kann, um sie auf den Daumen des Patienten einzustellen. Zudem erleichtert dies das An- und Ausziehen der Orthese (1).

Die flexible Schiene (113) hat die Möglichkeit, dem anatomischen Gelenk in Flexion (Beugung) und Extension (Streckung) zu folgen. Es begrenzt also nicht die Bewegungen des anatomischen Gelenks.

Durch die Beweglichkeit der erfindungsgemäßen Orthese (1) am Daumengrundgelenk (MCP) ist eine Haltung in jeder Stellung dieses Gelenks möglich. Dadurch wird ein Abbau des Kapsel-Band-Muskelapparat durch eine stetige, aber gleichzeitig schonende Beanspruchung verhindert.

Eine Beugung des Daumenendgelenks (IP) zur Entastung, wie in DE 20 2014 103 362 U1 beschrieben, ist mit der erfindungsgemäßen Orthese (1) nicht mehr notwendig, wodurch eine dauerhafte Entlastung des Gelenks gewährleistet wird.

Zudem kann je nach Schwere der Tätigkeit der Zug auf das Daumengrundgelenk (MCP) reduziert oder verstärkt werden. Außerdem wird das Daumengrundgelenk (MCP) nicht vollständig ruhiggestellt.

In einer bevorzugten Ausführungsform der erfindungsgemäßen Orthese (1) ist das Stellelement (115) dazu ausgelegt, die flexible Schiene (113) reversibel verschiebbar aufzunehmen, so dass der Abstand zwischen Schale (101) und Halterung (109) reversibel verstellbar ist.

Die Formulierung "reversibel verschiebbar" bedeutet, dass die flexible Schiene (113) so in dem Stellelement (115) gelagert ist, dass sie (in gewissen Grenzen) hinein und herausgeschoben werden kann, das heißt nach distal oder proximal verschoben werden kann, um so den Abstand zwischen Schale (101) und Halterung (109) reversibel zu verstellen. Auf diese Weise kann eine Zugkraft auf das Daumengrundgelenk (MCP) je nach Bedarf eingestellt werden.

Das Stellelement (115) übernimmt folglich die Aufgabe, die flexible Schiene (113) einzufahren und herauszuschieben und diese in einer bestimmten Position dann zu fixieren. Dieses Fixieren ist beispielsweise durch eine Art Schotklemme möglich, bei welcher manuell die Funktion ausgeschaltet werden kann, um ein leichtes An- und Ausziehen zu ermöglichen. Jedoch ist jede Art von Verschluss, welche die Aufgaben erfüllt anwendbar.

Durch diese Verstellung der im Bereich Handrückens angebrachten erfindungsgemäßen Orthese (1) kommt es zu einer geringen Spaltbildung des Daumensattelgelenks (CMP), da eine Spannung zwischen der am proximalen Handwurzelgelenk (RP) anliegenden Schale (101) mit Verschluss (103) und der Halterung (109) unterhalb des Daumenendgelenks (IP) angelegt wird. Dadurch wird ein Reiben der Gelenkflächen des Daumensattelgelenks (CMP) verhindert oder zumindest reduziert, was mit einer spürbaren Verringerung der Schmerzen einhergeht. Zudem bekommen die Gelenkflächen so die Möglichkeit, ein Ersatzgewebe zu bilden, welches eine neue Schutzschicht des Knochens darstellt. Dabei wird das Daumensattelgelenk (CMP) um bis zu 0,25 mm gestreckt.

Ein weiterer Vorteil ist, dass durch die weiterhin mögliche Bewegung der Gelenke die Produktion der Gelenkschmiere, lat. Synovia, angeregt wird, welche z.B. den restlichen vorhandenen Knorpel der Gelenkflächen mit Nährstoffen versorgt und als Gleitmittel dient.

Es ist in einer Weiterbildung der erfindungsgemäßen Orthese (1) bevorzugt, wenn das Stellelement (115) in Form einer Schotklemme oder als Schneckenradgetriebe ausgeführt ist.

Diese Ausführung stellt sicher, dass die im Stellelement (115) aufgenommene flexible Schiene (113) sehr einfach und praktikabel reversibel verstellt werden kann.

Aus therapeutischen Gründen kann es in einer Weiterbildung sinnvoll sein, wenn die flexible Schiene (113) durch deren eine Steifigkeit in Ihrem Winkelbereich begrenzbar ist. Dies ist durch die unterschiedlichen Steifigkeiten der flexiblen Schiene (113) möglich. Mit diesem Winkelbereich wird der Bewegungsbereich des Daumengrundgelenks (MCP) begrenzt.

Damit kann beispielsweise bei einer Hypermobilität des Daumengrundgelenks (MCP) die Flexion begrenzt werden, um weiterhin einen guten Kraftverlauf zu gewährleisten.

Die flexible Schiene (113) kann individuell oder konfektioniert an die jeweilige Größe der Hand des Patienten angepasst werden.

Da das Daumengrundgelenk (MCP) frei beweglich ist, ist es bevorzugt, diese freie Beweglichkeit in vollem Umfang dadurch sicherzustellen, dass das Stellelement (115) in Richtung des proximalen Handwurzelgelenks (RP) und in Richtung des Daumengrundgelenks (MCP) verstellbar ist und in jeder Position fixierbar ist, jedoch die Beugung (Flexion) und Streckung (Extension) durch die flexiblen Eigenschaften in vollem Umfang zulässt. Bei Bedarf kann auch eine in der Flexion (Beugung) und Extension (Streckung) begrenzende flexible Schiene mit einer höheren Steifigkeit benutzt werden, um den Bewegungsfreiraum einzugrenzen.

Eine spezielle Ausführungsform sieht vor, dass die Schale (101) mit der flexiblen Schiene (113) reversibel lösbar verbindbar ist.

Um ein Verrutschen der Schale (101) am proximalen Handwurzelgelenk (RP) zu vermeiden, weist die Schale (101) zumindest einen Druckpunkt distal des distalen Gelenkkopfs der Elle (CU), lat. Caput ulnae, oder distal des Griffelfortsatzes der Speiche (PSR), lat. Processus styloideus radii, auf. Der Druckpunkt kann insbesondere als Pelotte ausgebildet sein, die in die Schale (101) eingearbeitet ist.

Um ein Verrutschen der Schale (101) noch weiter zu verhindern, kann auch in dem Verschluss (103) ein Druckpunkt, insbesondere in Form einer Pelotte, vorgesehen sein, der distal des distalen Gelenkkopfs der Speiche (CR), lat. Caput radii, angeordnet ist. Ferner kann die Schale (101) mit rutschfesten Polstern versehen werden (z.B. aus Kautschuk).

Es ist bevorzugt, wenn die erfindungsgemäße Orthese (1) ferner eine Anzeigevorrichtung zur Überwachung einer Zugkraft aufweist. Hiermit kann einerseits sichergestellt werden, dass eine therapeutisch maximal zulässige Zugkraft nicht überschritten wird. Andererseits wird gewährleistet, dass der Patient nicht zu wenig Zugkraft ausübt und somit die Wirkung der erfindungsgemäßen Orthese (1) nicht oder nicht vollständig nutzt.

In einem zweiten Aspekt außerhalb der vorliegenden Erfindung wird eine Verwendung der erfindungsgemäßen Orthese (1) zur Ausübung einer Zugkraft auf das Daumensattelgelenk (CMP) und/oder auf das Daumengrundgelenk (MCP) zur Entlastung der Gelenkflächen dargestellt.

Wie vorstehend schon erwähnt, kann durch das Anlegen einer Zugkraft das Daumensattelgelenk (CMP) entlastet und die Bildung von Ersatzgewebe im Gelenk gefördert werden. Zudem kann direkt nach dem Anlegen der erfindungsgemäßen Orthese (1) und dem Einstellen des richtigen Zugs eine direkte Reduzierung der Schmerzen beim Patienten erreicht werden.

In einem dritten Aspekt außerhalb der vorliegenden Erfindung wird eine Verwendung der erfindungsgemäßen Orthese (1) zur Linderung von Beschwerden einer Erkrankung des Daumensattelgelenks (CMP) und des Daumengrundgelenks (MCP) aus dem degenerativen, entzündlichen und rheumatischen Formenkreis dargestellt.

Weitere Ziele, Merkmale, Vorteile und Anwendungsmöglichkeiten ergeben sich aus der nachfolgenden Beschreibung von die Erfindung nicht einschränkenden Ausführungsbeispielen anhand der Figuren. Dabei bilden alle beschriebenen und/oder bildlich dargestellten Merkmale für sich oder in beliebiger Kombination den Gegenstand der Erfindung, auch unabhängig von ihrer Zusammenfassung in den Ansprüchen oder deren Rückbeziehung. Es zeigen:
- Fig. 1:: eine schematische Darstellung einer menschlichen Hand von der Handinnenfläche her betrachtet mit der erfindungsgemäßen Orthese 1 und
- Fig. 2:: eine schematische Darstellung der in Figur 1 dargestellten menschlichen Hand vom Handrücken her betrachtet mit der erfindungsgemäßen Orthese 1.

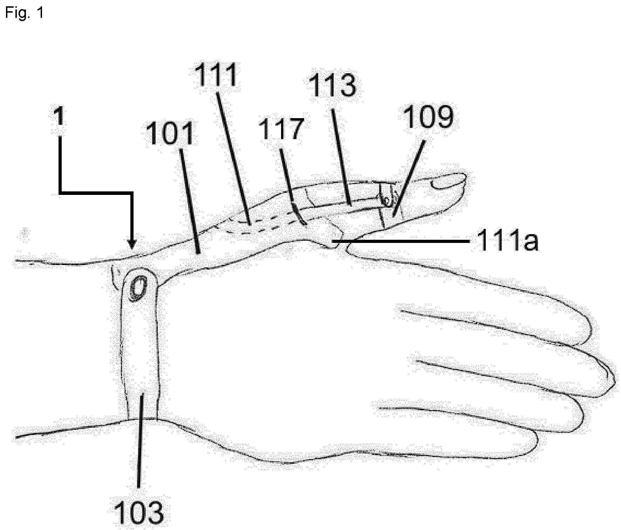

In Figur 1 wird eine menschliche Hand schematisch von der Handinnenfläche her (palmar) betrachtet dargestellt, wobei die erfindungsgemäße Orthese 1 angebracht ist. Die Schale 101 liegt auf dem Handballen auf und wird in dieser konkreten Ausführungsform mit dem Verschluss 103 von der dorsalen (rückseitigen), radialen (speichenseitig) und palmaren (handflächenseitigen) Seite am proximalen Handwurzelgelenk RP befestigt. Das Handgelenk wird somit zirkulär vollkommen umschlossen.

Der Verschluss 103 besteht hier aus einem Band, kann aber je nach Beschaffenheit und Bedarf auch variiert werden. Das Befestigen geschieht beispielsweise durch eine Haken-Ösen-Verbindung oder ein Klett-/Flauschband.

Die flexible Schiene 113 ist vom Handrücken aus kommend in einem Kanal 111 in der Schale 101 befestigt zu sehen, welche dann lateral/proximal den Kanal 111 am Kanal-Ausgang (117) verlässt und dann freilaufend nach distal läuft und dort parallel zum anatomischen Gelenk als eine Art mechanisches Gelenk fungiert. Alternativ zu dem Kanal 111 kann eine andere geeignete Halterung für die flexible Schiene 113 vorgesehen sein. Innerhalb des Kanals 111 verläuft die flexible Schiene 113 im Wesentlichen starr. Außerhalb des Kanals 111, ab dem Kanal-Ausgang (117) wird die Beweglichkeit der flexiblen Schiene 113 im Wesentlichen durch die Steifigkeit des verwendeten Materials bestimmt. In Relation zum Kanal 111 ist am Ende der Schale 101 ein Fixierpunkt 111a vorgesehen.

Am anderen Ende der Schiene 113 ist die Halterung 109 vorgesehen, die am distalen Ende des ersten Daumenknochens PPI ansetzt und diesen zirkulär vollkommen umschließt. Die Befestigung der Halterung 109 kann analog der Befestigung des Verschlusses 103 erfolgen.

In einer bevorzugten Ausführungsform weist die Halterung 109 eine U-förmige Halbschale auf, die aus einem der hier angegebenen Materialien gefertigt ist. Diese kann, entweder von dorsal oder palmar angebracht, zirkulär geschlossen werden. Die U-förmige Halbschale befindet sich unterhalb des Daumenendgelenks IP und gibt Druck unterhalb des ersten Daumenknochens PPI auf, so dass ein Verrutschen der U-förmigen Halbschale nach distal verhindert wird.

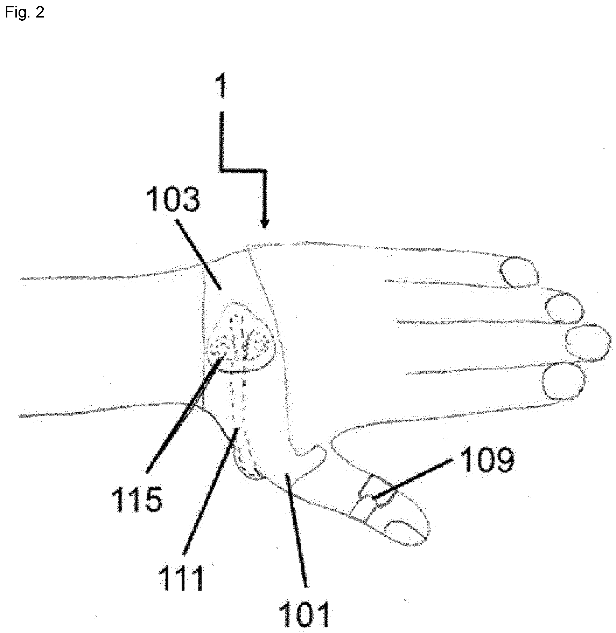

In Figur 2 im Bereich z.B. des Handrückens ist das Stellelement 115 zu erkennen, in das die flexible Schiene 113 durch einen vorliegenden Kanal 111 (oder eine andere passende Halterung) für die flexible Schiene 113 eingebracht ist. Am anderen Ende der flexible Schiene 113 ist die Halterung 109 vorgesehen, die am distalen Ende des ersten Daumenknochens PPI ansetzt und diesen in der gezeigten Ausführungsform zirkulär vollkommen umschließt. Die Befestigung der Halterung 109 kann analog der Befestigung des Verschlusses 103 erfolgen.

Die erfindungsgemäße Orthese 1 kann je nach Bedingungen aus unterschiedlichen Materialien gefertigt werden. Bevorzugt besteht sie aus einem erstarkten thermoplastischen Kunststoff, der das Handgelenk teilweise umschließt. Bevorzugt ist ein Niedertemperatur-Thermoplast, ein Faserverbundwerkstoff oder thermoplastischer Kunststoff.

Die flexible Schiene 113 wird bevorzugt aus einem Niedertemperatur-Thermoplast, einem Faserverbundwerkstoff oder einem thermoplastischen Kunststoff oder im 3D-Druckverfahren aus geeigneten Kunststoffen gefertigt.

In einer weiteren bevorzugten Ausgestaltung besteht die Schiene 101 aus einem gelochten Kunststoff, um der Haut das Atmen zu erleichtern.

### Bezugszeichenliste

- 1: Orthese
- 101: Schale
- 103: Verschluss
- 109: Halterung
- 111: Kanal
- 111a: Fixierpunkt
- 113: flexible Schiene
- 115: Stellelement
- 117: Kanal-Ausgang

- CMP: Daumensattelgelenk
- CR: Gelenkkopf der Speiche
- CU: Gelenkkopf der Elle
- IP: Daumenendgelenk
- MCP: Daumengrundgelenk
- PPI: erster Daumenknochen
- PSR: Griffelfortsatz der Speiche
- RP: proximales Handwurzelgelenk

## Patentansprüche

1. Orthese (1) für das Daumensattelgelenk (CMP), umfassend:
- eine Schale (101), die vom proximalen Handwurzelgelenk (RP) nach distal bis zum Daumengrundgelenk (MCP) verläuft, wobei die Schale (101) an ihrem distalen Ende sowohl offen als auch zirkulär vollkommen umschließend ausführbar ist,
- einen an der Schale (101) befestigten Verschluss (103) im Bereich des proximalen Handwurzelgelenks (RP), welcher am proximalen Handwurzelgelenk (RP) zirkulär vollkommen umschließend fixierbar ist,
- einen in der Schale (101) vorgesehenen Kanal (111), welcher mit dem Kanal-Ausgang (117) endet,
- eine flexible Schiene (113), die teilweise in dem Kanal (111) von der Schale (101) bis zum Daumenendgelenk (IP) verläuft und diesen durch den Kanal-Ausgang (117) verlässt,
- eine Halterung (109), die an einem ersten Ende der flexiblen Schiene (113) vorgesehen ist und am distalen Ende des ersten Daumenknochens (PPI) zirkulär vollkommen umschließend fixierbar ist, und
- ein Stellelement (115), das an der Schale (101) angeordnet ist und das die flexible Schiene (113) an deren zweitem Ende reversibel aufnimmt,
**dadurch gekennzeichnet, dass** die flexible Schiene (113) von dem Stellelement (115) durch den Kanal (111) verläuft und diesen lateral/proximal des Daumengrundgelenks (MCP) verlässt, wobei die flexible Schiene (113) im Bereich des Kanal-Ausgangs (117) parallel zum anatomischen Gelenkdrehpunkt verläuft, wobei die flexible Schiene (113) die Schale (101) mit der Halterung (109) reversibel lösbar verbindet.

2. Orthese (1) nach Anspruch 1, wobei das Stellelement (115) dazu ausgelegt ist, die flexible Schiene (113) reversibel verschiebbar aufzunehmen, so dass der Abstand zwischen Schale (101) und Halterung (109) reversibel verstellbar ist.

3. Orthese (1) nach Anspruch 1 oder 2, wobei das Stellelement (115) in Form einer Schotklemme oder als Schneckenradgetriebe ausgeführt ist.

4. Orthese (1) nach einem der Ansprüche 1 bis 3, wobei die flexible Schiene (113) durch deren eine Steifigkeit in ihrem Winkelbereich begrenzbar ist.

5. Orthese (1) nach einem der Ansprüche 1 bis 4, wobei das Stellelement (115) in Richtung des proximalen Handwurzelgelenks (RP) und in Richtung des Daumengrundgelenks (MCP) verstellbar ist und in jeder Position fixierbar ist.

6. Orthese (1) nach einem der Ansprüche 1 bis 5, wobei die Schale (101) mit der flexiblen Schiene (113) reversibel lösbar verbindbar ist.

7. Orthese (1) nach einem der Ansprüche 1 bis 6, wobei die Schale (101) zumindest einen Druckpunkt distal des distalen Gelenkkopfs der Elle (CU) oder distal des Griffelfortsatzes der Speiche (PSR) aufweist.

8. Orthese (1) nach einem der Ansprüche 1 bis 7, wobei diese ferner eine Anzeigevorrichtung zur Überwachung einer Zugkraft aufweist.

## Claims

1. Orthosis (1) for the thumb saddle joint (CMP), comprising
- a shell (101) that extends from the proximal wrist joint (RP) distally to the thumb basal joint (MCP), wherein the shell (101) can be designed to be completely encircling at its distal end (in the area of the metacarpophalangeal joint) as well as open,
- a closure (103) in the region of the proximal wrist joint (RP), which is attached to the shell (101) and can be fixed to the proximal wrist joint (RP) in a completely encircling manner,
- a channel (111) provided in the shell (101), which ends with the channel outlet (117),
- a flexible splint (113), which extends partially in the channel (111) from the shell (101) to the thumb end joint (IP) and leaves the latter through the channel outlet (117),
- a holder (109) which is provided at a first end of the flexible splint (113) and can be fixed at the distal end of the first thumb bone (PPI) in a completely encircling manner, and
- an adjusting element (115) which is arranged on the shell (101) and which reversibly receives the flexible splint (113) at its second end,
**characterised in that** the flexible splint (113) extends from the adjusting element (115) through the channel (111) and leaves the latter laterally/proximal to the thumb basal joint (MCP), wherein the flexible splint (113) extends parallel to the anatomical pivot point of the joint in the region of the channel outlet (117) and the flexible splint (113) reversibly detachably connects the shell (101) to the holder (109).

2. Orthosis (1) according to claim 1, wherein the adjusting element (115) is designed to reversibly displaceably receive the flexible splint (113) so that the distance between the shell (101) and the holder (109) is reversibly adjustable.

3. Orthosis (1) according to claim 1 or 2, wherein the adjusting element (115) is designed in the form of a jam cleat or as a worm gear.

4. Orthosis (1) according to one of claims 1 to 3, wherein the flexible splint (113) can be limited in its angular range by its rigidity.

5. Orthosis (1) according to one of the claims 1 to 4, wherein the adjusting element (115) is adjustable in the direction of the proximal wrist joint (RP) and in the direction of the metacarpophalangeal joint (MCP) and is fixable in each position.

6. Orthosis (1) according to one of the claims 1 to 5, wherein the shell (101) is reversibly releasably connectable to the flexible splint (113).

7. Orthosis (1) according to one of the claims 1 to 6, wherein the shell (101) has at least one pressure point distal to the distal joint head of the ulna (CU) or distal to the styloid process of the radius (PSR).

8. Orthosis (1) according to one of the claims 1 to 7, wherein it further has an indicator for monitoring a tensile force.

## Revendications

1. Orthèse (1) pour l'articulation carpo-métacarpienne du pouce (CMP), comprenant :
- une coque (101), qui s'étend depuis l'articulation carpienne proximale (RP) distalement jusqu'à l'articulation métacarpo-phalangienne (MCP), dans laquelle la coque (101) peut être réalisée, sur son extrémité distale, à la fois de manière ouverte et de manière totalement enveloppante de manière circulaire,
- une fermeture (103) fixée sur la coque (101) dans la zone de l'articulation carpienne proximale (RP), laquelle peut être fixée de manière totalement enveloppante de manière circulaire sur l'articulation carpienne proximale (RP),
- un canal (111) prévu dans la coque (101), lequel se termine avec la sortie de canal (117),
- un rail flexible (113), qui s'étend en partie dans le canal (111) depuis la coque (101) jusqu'à l'articulation terminale du pouce (IP) et quitte celle-ci par la sortie de canal (117),
- un support (109), qui est prévu sur une première extrémité du rail flexible (113) et qui peut être bloqué de manière totalement enveloppante de manière circulaire sur l'extrémité distale du premier os du pouce (PPI), et
- un élément de réglage (115), qui est disposé sur la coque (101) et qui reçoit le rail flexible (113) sur sa seconde extrémité,
**caractérisée en ce que** le rail flexible (113) s'étend depuis l'élément de réglage (115) à travers le canal (111) et quitte celui-ci latéralement/proximalement par rapport à l'articulation métacarpo-phalangienne (MCP), dans laquelle le rail flexible (113) s'étend, dans la zone de la sortie de canal (117) de manière parallèle au pivot anatomique d'articulation, dans laquelle le rail flexible (113) relie de manière amovible et réversible la coque (101) au support (109).

2. Orthèse (1) selon la revendication 1, dans laquelle l'élément de réglage (115) est conçu pour recevoir de manière à pouvoir coulisser de manière réversible le rail flexible (113) de telle sorte que la distance entre la coque (101) et la fixation (109) peut être ajustée de manière réversible.

3. Orthèse (1) selon la revendication 1 ou 2, dans laquelle l'élément de réglage (115) est réalisé sous la forme d'une pince à feuille ou d'un engrenage à vis sans fil.

4. Orthèse (1) selon l'une quelconque des revendications 1 à 3, dans laquelle le rail flexible (113) peut être limité dans sa plage angulaire par sa rigidité.

5. Orthèse (1) selon l'une quelconque des revendications 1 à 4, dans laquelle l'élément de réglage (115) peut être ajusté en direction de l'articulation carpienne proximale (RP) et en direction de l'articulation métacarpo-phalangienne (MCP) et peut être fixé dans chaque position.

6. Orthèse (1) selon l'une quelconque des revendications 1 à 5, dans laquelle la coque (101) peut être reliée de manière amovible et réversible au rail flexible (113).

7. Orthèse (1) selon l'une quelconque des revendications 1 à 6, dans laquelle la coque (101) présente au point un point de pression distalement par rapport au condyle distal du cubitus (CU) ou distalement par rapport à l'apophyse styloïde du radius (PSR).

8. Orthèse (1) selon l'une quelconque des revendications 1 à 7, dans laquelle celle-ci présente en outre un dispositif d'affichage destiné à surveiller une force de traction.
